(19) Europäisches Patentamt
European Patent Office
Office européen des brevets

(11) Veröffentlichungsnummer: **0 055 851**
**A1**

(12) # EUROPÄISCHE PATENTANMELDUNG

(21) Anmeldenummer: **81110772.1**

(22) Anmeldetag: **24.12.81**

(51) Int. Cl.³: **C 07 D 209/52**
A 61 K 31/40
//C07D409/06, C07D409/12

(30) Priorität: **03.01.81 DE 3100083**

(43) Veröffentlichungstag der Anmeldung:
**14.07.82 Patentblatt 82/28**

(84) Benannte Vertragsstaaten:
**AT BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Bartmann, Wilhelm, Dr.**
**Am Dachsbau 5**
**D-6232 Bad Soden am Taunus(DE)**

(72) Erfinder: **Beck, Gerhard, Dr.**
**Gustav-Freytag-Strasse 24**
**D-6000 Frankfurt am Main(DE)**

(72) Erfinder: **Knolle, Jochen, Dr.**
**Höchster Strasse 21**
**D-6239 Kriftel(DE)**

(72) Erfinder: **Rupp, Richard Helmut, Dr.**
**Röderweg 16a**
**D-6240 Königstein/Taunus(DE)**

(54) **11-Desoxy-hetero-imino-prostacycline, Verfahren zu ihrer Herstellung und ihre Verwendung als Arzneimittel.**

(57) Gegenstand der vorliegenden Erfindung sind neue Analoga des $PGI_2$ der allgemeinen Formel I

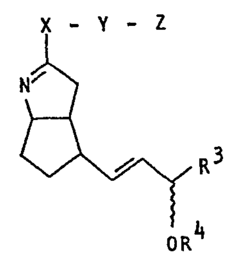

(1)

die eine spezifischere Wirkung und/oder eine längere Wirkungsdauer als $PGI_2$ besitzen, Verfahren zu ihrer Herstellung sowie Arzneimittel, die solche $PGI_2$-Analoge als Wirkstoffe enthalten.

EP 0 055 851 A1

Croydon Printing Company Ltd.

# 11-Desoxy-hetero-imino-prostacycline, Verfahren zu ihrer Herstellung und ihre Verwendung als Arzneimittel

Prostacyclin $PGI_2$, ein kürzlich isolierter Naturstoff aus der Familie der Prostaglandine, zeichnet sicht durch seine stark ausgeprägten thrombocytenaggregationshemmenden Eigenschaften aus (The Lancet 1977, 18). Außerdem vermag $PGI_2$ einige Blutgefäße, z.B. Coronararterien zu relaxieren (Prostaglandins 13, 3, 1977), so daß es zur Therapie und Prophylaxe von Thrombosen und Infarkten Verwendung finden kann. $PGI_2$ zeigt weiter eine ausgeprägte blutdrucksenkende Wirkung (z.B. IRCS Med. Sci. 6, 392 (1978)).

Gegenstand der vorliegenden Erfindung sind neue Analoga des $PGI_2$ der allgemeinen Formel I

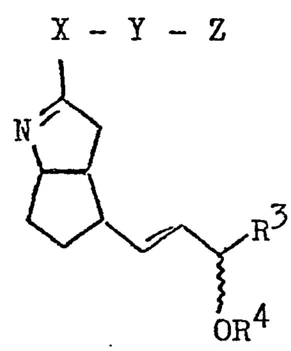

(I)

die eine spezifischere Wirkung und/oder eine längere Wirkungsdauer als $PGI_2$ besitzen und in welcher bedeuten:

X ein Sauerstoff- oder Schwefelatom oder eine NH-Gruppe;

Y einen geradkettigen oder verzweigten Alkylenrest mit bis zu 8 Kohlenstoffatomen oder einen geradkettigen oder verzweigten ungesättigten aliphatischen Rest mit 3 bis 8 Kohlenstoffatomen oder einen cycloaliphatischen Rest mit 3 bis 6 Kohlenstoffatomen oder einen Phenylenrest;

Z einen Rest der Formel $-CO_2R^1$, $-CH_2OH$ oder $CH_2-N\ (R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten

aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder ein Ammoniumion, das sich von einem primären, sekundären oder tertiären Amin ableitet, oder ein Tetraalkylammonium-Ion,

$R^2$ Wasserstoff oder einen geradkettigen oder verzweigten aliphatischen Kohlenwasserstoffrest mit bis zu 5 C-Atomen oder $R^2$-$R^2$ zusammen auch eine $-(CH_2)_n-$ Gruppe mit n = 3 bis 6,

$R^3$ einen Arylrest, der im Kern 1- bis 3-fach substituiert sein kann mit Halogen, Trifluormethyl und/oder Alkyl oder Alkoxy mit je 1 bis 6 C-Atomen,
oder einen cycloaliphatischen Rest mit 3 bis 8 Kohlenstoffatomen oder
einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, die ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Phenyl oder einem α- oder ß-Thienyl- oder α- oder ß-Furylrest, die ihrerseits im Kern 1- bis 3-fach substituiert sein können mit Halogen, Trifluormethyl und/oder Alkyl oder Alkoxy mit je 1 bis 6 C-Atomen,

c) einem Phenoxy-, einem α- oder ß-Thienyloxyrest oder einem Cycloalkoxyrest mit 3 bis 7 Kohlenstoffatomen, wobei die genannten Reste ihrersseits im Kern 1- bis 3-fach substituiert sein können mit Halogen, Trifluormethyl und/oder Alkyl oder Alkyloxy mit je 1 bis 6 C-Atomen,

$R^4$ Wasserstoff oder eine unter neutralen oder basischen Bedingungen leicht abspaltbare Schutzgruppe.

Unter den Substituenten $R^1$ sind bevorzugt:
Wasserstoff, geradkettiges oder verzweigtes Alkyl mit bis zu 8 C-Atomen, ein geradkettiger oder verzweigter ungesättigter aliphatischer Kohlenwasserstoffrest mit bis zu 4 C-Atomen, ein cycloaliphatischer Kohlenwasserstoffrest mit 5 bis 7 C-Atomen, ein araliphatischer Kohlenwasserstoffrest mit bis zu 9 C-Atomen oder ein Ammoniumion, das sich von einem primären, sekundären oder tertiären Amin ableitet, insbesondere:

Wasserstoff, Methyl, Ethyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl, Methylammonium, Dicyclohexylammonium, Tris-(hydroxymethylammonium).

Unter den Substituenten $R^3$ sind die im folgenden aufgeführten besonders bevorzugt:
unsubstituiertes Phenyl oder mit Halogen, Trifluormethyl, $C_{1-4}$-Alkyl oder $C_{1-4}$-Alkoxy einfach substituiertes Phenyl, geradkettiges oder verzweigtes $C_{3-7}$-Alkyl, das substituiert sein kann mit gegebenenfalls substituierten $C_{5-7}$-Cycloalkyl, mit $C_{1-3}$-Alkoxy, mit Phenoxy oder Halogenphenoxy, mit Thienyloxy oder Halogenthienyloxy, mit Cyclohexyloxy, mit Thienyl, mit Halogenthienyl oder mit Furyl, insbesondere:
n-Pentyl, 1,1-Dimethylpentyl, Cyclopentylmethyl, Cyclohexylmethyl, 1,1-Dimethyl-2-ethoxy-ethyl, 1,1-Dimethyl-2-methoxyethyl, 1,1-Dimethyl-cyclohexyloxymethyl, 1-Fluorpentyl, 1-Chlor-pentyl, 5-Fluorpentyl, 5-Chlorpentyl, 3-Thienyl-2-ethyl, 2-Thienyl-2-ethyl, 3-(2-Chlorthienyl)-2-ethyl, 2-(5-Chlor-thienyl)-2-ethyl, Phenoxymethyl, 3-Chlor-phenoxymethyl, 2-Thienyloxymethyl, 3-(2-Chlorthienyl)-oxy-methyl, 2-(5-Chlor-thienyl)-oxymethyl, 3-Furyl-2-ethyl, 2-Furyl-2-ethyl, 2,2,3,3-Tetrafluorcyclobutyl-2-ethyl, Phenyl, 3-Chlor-phenyl,

3-Trifluormethylphenyl.

Unter den Substituenten Y sind die folgenden aufgeführten besonders bevorzugt:
Ethyliden, Trimethylen, Methyltrimethylen, Methyltetramethylen, Propenylen, Cyclobutenylen, Cyclopentenylen, Phenylen.

Gegenstand der Erfindung ist außerdem ein Verfahren zur Herstellung von Prostacyclinderivaten der Formel I, das dadurch gekennzeichnet ist, daß man

a) den Benzylether der Formel II

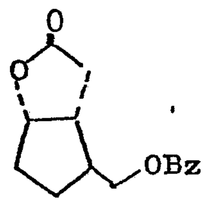

(II)

worin Bz den Rest $CH_2$ 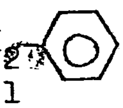 bedeutet, mit Ammoniak zum Hydroxyamid der Formel

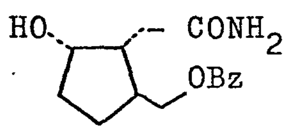

(III)

umsetzt,

b) das Hydroxyamid der Formel III zum Ketoamid der Formel IV oxidiert

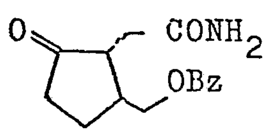

(IV)

welches im tautomeren Gleichgewicht mit dem Hydroxylactam der Formel V

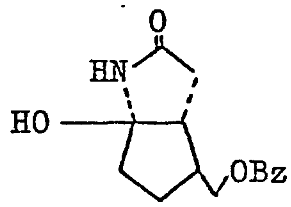

(V)

steht,

c) das Hydroxylactam der Formel V, das Ketoamid der Formel IV oder eine Mischung aus Hydroxylactam der Formel V und Ketoamid der Formel IV mit einem Mercaptan der Formel VI

$$R^5 - SH \qquad (VI)$$

worin $R^5$ einen Alkylrest mit 1 bis 5 C-Atomen oder einen Phenylrest bedeutet, umsetzt zu einem Thioether der Formel VII

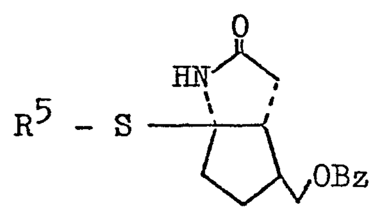

(VII)

worin $R^5$ die zur Formel VI gegebenen Bedeutungen hat,

d) im Thioether der Formel VII den Rest $-SR^5$ durch Reduktion abspaltet, wobei ein Lactam der Formel VIII

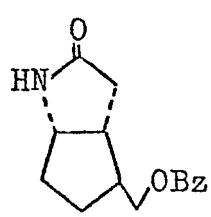

(VIII)

erhalten wird,

e) die Benzylethergruppe im Lactam der Formel VIII abhydriert, wobei ein Hydroxylacatam der Formel IX

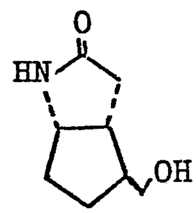

(IX)

erhalten wird,

f) den Alkohol der Formel IX zum Aldehyd der Formel X

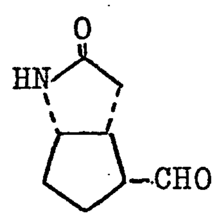

(X)

oxidiert,

g) den Aldehyd der Formel X mit einem Phosphat der Formel XI

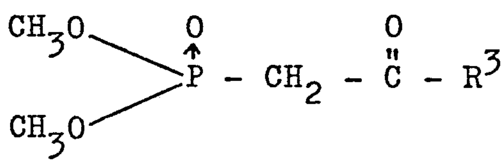

(XI)

worin $R^3$ die zur Formel I gegebenen Bedeutungen hat, zu einem Enon der Formel XII

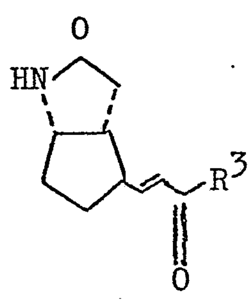

(XII)

umsetzt,

h) das Enon der Formel XII in bekannter Weise mit einem geeigneten Reduktionsmittel zu einem Epimerengemisch der

Alkohole der Formel XIII

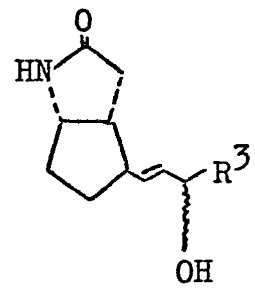

(XIII)

worin $R^3$ die zur Formel I gegebenen Bedeutungen hat, reduziert,

i) die Alkoholfunktion in den Alkoholen der Formel XIII durch eine Gruppe schützt, die unter neutralen oder basischen Bedingungen wieder abgespalten werden kann, wodurch eine Verbindung der Formel XIV

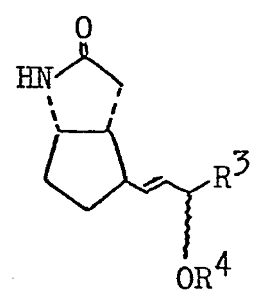

(XIV)

worin $R^3$ die zur Formel I gegebenen Bedeutungen hat, und $R^4$ eine unter neutralen oder basischen Bedingungen leicht abspaltbare Schutzgruppe bedeutet, erhalten wird,

j) das Lactam der Formel XIV durch übliche Methoden in ein Thiolactam der Formel XV

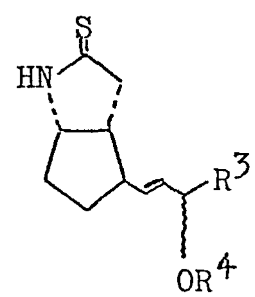

(XV)

worin $R^3$ die zur Formel I, $R^4$ die zur Formel XIV gegebene Bedeutung hat, überführt,

k) das Thiolactam der Formel XV mit einem Alkylhalogenid der Formel XVI

$$Z - Y - Hal \qquad (XVI)$$

worin Hal Jod, Chlor oder Brom bedeutet und Y und Z die zur Formel I gegebenen Bedeutungen haben, zu einem Thiolactimether der Formel I

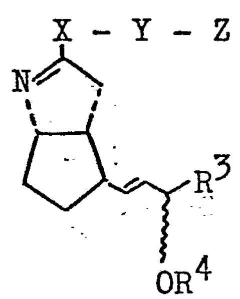

(I)

alkyliert,
worin Y, Z und $R^3$ die zur Formel I und $R^4$ die zur Formel XIV gegebene Bedeutung haben und X ein Schwefelatom bedeutet, oder

l) das Lactam der Formel XIV mit einem Alkylhalogenid der Formel XVI zu einem Lactimether der Formel I

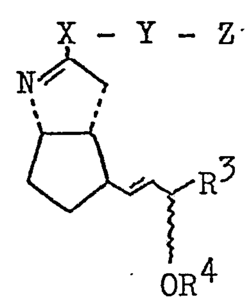

(I)

alkyliert,
worin Y, Z und $R^3$ die zur Formel I und $R^4$ die zur

Formel XIV gegebene Bedeutung haben und X ein Sauerstoffatom bedeutet, oder

m) ein Thiolactam der Formel XV in ein Alkylthiolactimether der Formel XVII überführt

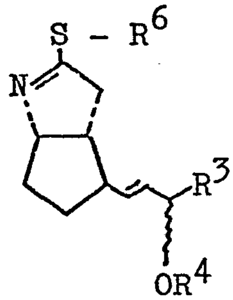

(XVII)

worin $R^3$ die zur Formel I und $R^4$ die zur Formel XIV gegebene Bedeutung hat und $R^6$ einen $C_{1-4}$-Alkylrest bedeutet,

n) den Thiolactimether der Formel XVII mit einem Amin der Formel XVIII

$$Z - Y - NH_2 \qquad (XVIII)$$

worin Z und Y die zur Formel I gegebenen Bedeutungen haben, zu einem Amidin der Formel I

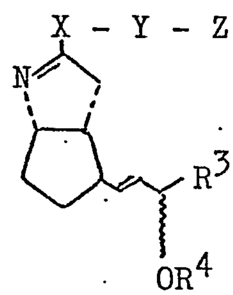

(I)

worin Y, Z und $R^3$ die zur Formel I und $R^4$ die zur Formel XIV gegebene Bedeutung hat, und X den NH-Rest darstellt, umsetzt,

o) gegebenenfalls in einer Verbindung der Formel I die Schutzgruppe $R^4$ durch basisch katalysierte Hydrolyse

abspaltet,

p) gegebenenfalls einer Verbindung der Formel I, in welcher Z den Rest $CO_2R^1$ bedeutet, wobei $R^1$ den obengenannten Rest darstellt, jedoch nicht Wasserstoff oder ein Kation bedeutet, und $R^4$ Wasserstoff bedeutet, zu einer Verbindung der Formel I verseift, in welcher Z den Rest $CO_2R^1$ darstellt, wobei $R^1$ Wasserstoff oder ein physiologisch verträgliches Kation bedeutet,

q) gegebenenfalls eine Verbindung der Formel I, in welcher Z den Rest $CO_2R^1$ darstellt, wobei $R^1$ Wasserstoff oder ein Kation bedeutet, zu einer Verbindung der Formel I verestert, worin Z den Rest $CO_2R^1$ darstellt, wobei $R^1$ die zur Formel I genannten Bedeutungen hat, jedoch nicht Wasserstoff oder ein Kation bedeutet, und

r) gegebenenfalls in einer Verbindung der Formel I, in welcher Z den Rest $CO_2R^1$ darstellt, wobei $R^1$ ein physiologisch verträgliches Kation darstellt, dieses Kation gegen ein anderes austauscht.

Für die Herstellung des im erfindungsgemäßen Verfahren als Ausgangsmaterial verwendeten Alkohols II kann man analog dem Verfahren arbeiten, das in J. Org. Chem. 39, 256 (1974) beschrieben ist.

Das Lacton der Formel II läßt sich mit Ammoniak, z.B. in alkoholischer Lösung bei erhöhter Temperatur (70 bis 150°C) zum Hydroxyamid der Formel III umsetzen. Die Reaktion kann auch in Gegenwart von Katalysatoren, wie z.B. 2-Pyridon, Natriumamid oder Natriumalkoholaten durchgeführt werden. Zweckmäßigerweise arbeitet man im Autoklaven.

Die Oxidation des so erhaltenen Hydroxamids der Formel III zum Ketoamid der Formel IV kann mit Oxidationsmitteln wie Chromtrioxid/Dimethylsulfat, Chromdioxid/Pyridin, Pyridiniumchlorochromat, Pyridiniumdichromat, Chromtrioxid/Schwefelsäure/Wasser in inerten Lösungsmitteln wie Dimethylformamid, Methylenchlorid oder Aceton bei Temperaturen von -30°C bis 40°C, bevorzugt zwischen -25°C und -20°C, durchgeführt werden. Man kann jedoch auch mit "aktiviertem" Dimethylsulfoxid oxidieren (Tetrahedron 34, 1651 (1978)). Das Ketoamid der Formel IV kann an dieser Stelle durch Chromatographie an Kieselgel gereinigt werden.

Das Ketoamid der Formel IV steht im tautomeren Gleichgewicht mit seinem cyclischen Isomeren, dem Hydroxylactam der Formel V. Solche Gleichgewichte sind aus der Literatur bekannt, z.B. Chem. Ber. 103, 3205 (1970).

Löst man das Ketoamid der Formel IV in einem inerten Lösungsmittel, z.B. Aceton, Methanol, Ethanol, Tetrahydrofuran, Chloroform oder Methylenchlorid und läßt die Lösung bei 20°C bis 40°C stehen, erhält man das Hydroxylactam der Formel V.

Das Hydroxylactam der Formel V, das Ketoamid der Formel IV oder Mischungen aus beiden lassen sich mit Mercaptanen der Formel VI und Chlortrimethylsilan in interten Lösungsmitteln, wie z.B. Methylenchlorid, Chloroform, Toluol oder Dimethoxyethan zu Thioethern der Formel VII umsetzen. Die Reaktion wird bei Temperaturen von 30°C bis 100°C durchgeführt, zweckmäßigerweise in Gegenwart einer organischen Base, wie z.B. Pyridin, Triethylamin oder 1,4-Diazabicyclo-/4,3,0/nonen-5 (DBN).

Die Reduktion der Thioether der Formel VII erfolgt durch Umsetzung mit Wasserstoff in Gegenwart eines Metallkatalysators wie z.B. Pd/C, Raney-Ni oder $NiCl_2/NaBH_4$ in einem niedrig-Alkanol, wie z.B. Methanol, Ethanol, t-Butanol oder iso-Propanol oder in Aceton.

Durch Erwärmen erhält man den Benzylether der Formel VIII.

Der Benzylether der Formel VIII läßt sich mit Pd/C und Wasserstoff in einem niedrig-Alkanol, z.B. Methanol, i-Propanol oder Butanol, in THF oder Essigester zum Alkohol der Formel IX umsetzen. Dabei können dem Lösungsmittel 5 bis 10 % einer Mineralsäure, z.B. konz. Chlorwasserstoffsäure, oder einer organischen Säure wie z.B. Essigsäure zugesetzt werden. Man kann dabei bei Normaldruck und Raumtemperatur arbeiten oder die Reaktion im Autoklaven bei 20 bis 80°C unter einem Druck von 50 bis 100 atm. durchführen.

Die Oxidation des Alkohols der Formel IX zum Aldehyd der Formel X kann durch Oxidationsmittel wie Pyridiniumchlorochromat, Pyridiniumdichromat in inerten Lösungsmitteln wie Methylenchlorid oder Chloroform erfolgen. Weiter kann diese Oxidation auch durch "aktiviertes" Dimethylsulfoxid erfolgen. Eine bevorzugte Ausführungsform besteht darin, daß man eine Lösung des Alkohols der Formel IX in einer Mischung aus Methylenchlorid und Dimethylsulfoxid bei -60°C zu einer Lösung von Dimethylsulfoxid, das durch Oxalylsäurechlorid aktiviert wurde, in Methylenchlorid tropft. Der nach Aufarbeitung erhaltene Aldehyd der Formel X wird im allgemeinen ohne weitere Reinigung nach Horner-Emmon-Wittig mit einem Phosphonsäureester der Formel XI zu einem ungesättigten Keton der Formel XII umgesetzt, wobei eine bevorzugte Ausführungsform darin besteht, daß man das Natriumsalz des Phosphonsäureesters der Formel XI mit Natriumhydrid in Dimethoxyethan herstellt und anschließend den Aldehyd der Formel X zugibt und bei Raumtemperatur 2 bis 6 Stunden reagieren läßt. Die Phosphonsäureester der Formel XI werden nach literaturbekannten Verfahren (s. z.B. J. Am. Chem. Soc. 88, 5654 (1966)) hergestellt.

Die Alkohole der Formel XIII erhält man in Form ihrer Epimerengemische, wenn man die Ketone der Formel XII mit einem komplexen Metallhydrid, vorzugsweise einem Alkaliboranat oder mit D,L-Isobornyloxyaluminium-isopropoxid reduziert.

Der Schutz der Alkoholfunktion in Verbindungen der Formel XII erfolgt durch Umsetzung entweder mit Anhydriden der Formel $R^4$-O-$R^4$ oder Säurechloriden der Formel $R^4$-Cl, wobei $R^4$ z.B. eine aliphatische oder aromatische Acylgruppe bedeutet. Unter den Acylgruppen sind die im folgenden aufgeführten Substituenten bevorzugt:

$C_{2-4}$-Alkanoyl, insbesondere Acetyl, Propionyl, Benzoyl, sub. Benzoyl (z.B. 3-Methyl-benzoyl, 4-Phenylbenzoyl, 2,4-Dinitrobenzoyl, 2-Nitrobenzoyl), 1-Naphtholyl, 2-Naphtholyl. Die Reaktion wird im Falle der Anhydride ohne Lösungsmittel in Gegenwart einer Base oder im Falle der Umsetzung mit Säurechloriden in inerten Lösungsmitteln wie z.B. Chloroform, Methylenchlorid, Tetrachlorkohlenstoff, Tetrahydrofuran oder Dioxan in Gegenwart einer Base durchgeführt. Als Basen eignen sich z.B. Pyridin, Triethylamin oder 4-Dimethylaminopyridin.

Man kann aber auch Silylschutzgruppen verwenden. Dazu wird der Alkohol der Formel XIII in einem inerten Lösungsmittel, wie z.B. Chloroform, Methylenchlorid oder Toluol bei 0 bis 30°C mit dem Silylchlorid, bevorzugt Dimethyl-t-butylsilylchlorid, und einer Base zur Reaktion gebracht. Als Basen eignen sich z.B. Triethylamin, Pyridin oder Imidazol.

Die Thiolactame der Formel XV lassen sich aus den Lactamen der Formel XIV durch Umsetzung mit schwefelübertragenden Reagentien, wie z.B. Phosphorpentasulfid, Phosphorpentasulfid/Calciumoxid, Phosphorpentasulfid-Pyridin-Komplex oder Phosphorpentasulfid-Anisol-Komplex in inerten Lösungsmitteln, wie z.B. Toluol, Dimethoxyethan oder Pyridin nach literaturbekannten Methoden herstellen (s. z.B. Bull. Soc. Chim. Belg. 87, (3), 229 (1978)).

Zur Darstellung von Thiolactimethern der Formel I werden die Thiolactame der Formel XV mit Alkylhalogeniden der Formel XVI alkyliert. Diese Reaktion wird in einem inerten

Lösungsmittel, wie z.B. Toluol, Tetrahydrofuran, Dimethoxyethan oder Dimethylformamid in Gegenwart von Basen, wie z.B. Pyridin, Triethylamin, Kaliumcarbonat oder Natriumhydrid bei 15 bis 55°C durchgeführt. Eine bevorzugte Ausführungsform dieser Reaktion besteht darin, daß man das Natriumsalz der Thiolactame der Formel XV mit Natriumhydrid in Dimethoxyethan herstellt, und dazu eine Lösung des Alkylhalogenids der Formel XVI in Dimethoxyethan tropft und 2 bis 6 Stunden bei 25°C rührt.

Die Darstellung von Lactimethern der Formel I erfolgt durch Alkylierung der Lactame der Formel XIV mit Alkylhalogeniden der Formel XVI in einem inerten Lösungsmittel wie Benzol, Toluol, Dioxan oder Xylol bei Temperaturen von 80 bis 160°C in Gegenwart einer anorganischen Base, bevorzugt Silberoxid oder Silberhydroxid.

Zur Darstellung der Amidine der Formel I wird das Thiolactam der Formel XV zu einer S-Alkylverbindung der Formel XVII alkyliert. Dabei kann man wie bei der Herstellung der Thiolactimether der Formel I arbeiten, indem man zunächst mit Natriumhydrid in einem inerten Lösungsmittel wie Dimethoxyethan, Tetrahydrofuran oder Dioxan das Natriumsalz des Thiolactams herstellt und dann mit einem Alkylhalogenid der Formel $R^6$-Halogen, wobei $R^6$ Methyl oder Ethyl und Halogen, Jod, Brom oder Chlor, bevorzugt aber Jod bedeutet, umsetzt. Man kann aber auch das Thiolactam der Formel XV mit dem Alkylhalogenid der Formel $R^2$-Halogen in einem inerten Lösungsmittel wie Aceton, Essigester oder Chloroform umsetzen, und den Thiolactimether der Formel XVII mit Basen wie Natriumhydrogencarbonat, Natrium- oder Kaliumcarbonat freisetzen.

Durch Erhitzen der Thiolactimether der Formel XVII mit Aminen der Formel XVIII in einem Niedrigalkanol, wie z.B. Methanol oder Ethanol oder in inerten Lösungsmitteln wie Dioxan oder Aceton erhält man die Amidine der Formel I.

Die Schutzgruppe $R^4$ in einer Verbindung der Formel I kann unter milden alkalischen Bedingungen, z.B. mit Natrium- oder Kaliumcarbonat in alkoholischer Lösung abgespalten werden. Man arbeitet bei -10 bis +30°C.

Verbindungen der Formel I, worin Z einen Rest der Formel $-CO_2R^1$ darstellt und $R^1$ nicht Wasserstoff oder ein Kation bedeutet, können in üblicher Weise in alkalischem Medium zu Verbindungen der Formel I, worin Z eine Carboxylgruppe bedeutet, verseift werden, z.B. mit NaOH oder KOH in einem niedermolekularen Alkohol wie Methanol oder Ether wie Dimethoxyether oder THF, gegebenenfalls in Gegenwart von Wasser. Vorteilhafterweise benutzt man die äquimolare Menge oder einen sehr geringen Überschuß an Alkalihydroxid, so daß man das Alkalisalz der Formel I ($R^1$ = Alkalimetallion) durch Verdampfen des Lösungsmittels erhält, vorzugsweise durch Gefriertrocknung.

Das Alkalikation läßt sich an Ionenaustauschern in üblicher Weise gegen beliebige Kationen austauschen. Dazu läßt man die Lösung des Alkalisalzes eines erfindungsgemäßen 11-Deoxy-Hetero-Imino-Prostacyclinderivates durch eine mit einem Kationenaustauscher, wie z.B. Amberlite CG-50 oder Dowex CCR-2 gefüllte Säule laufen.

Der Kationenaustauscher ist mit dem erwünschten Kation beladen, z.B. mit einem Ammonium, das sich von einem primären, sekundären oder tertiären Amin ableitet. Das erwünschte Salz erhält man durch Eindampfen des Eluats.

Man kann Verbindungen der Formel I, bei denen Z einen Rest der Formel $-CO_2R^1$ und $R^1$ = $NH_4$ oder ein Ammoniumion bedeutet, das sich von einem primären, sekundären oder tertiären Amin ableitet, auch herstellen, indem man Verbindungen der Formel I, bei denen Z Carboxyl und $R^1$ = Wasserstoff bedeutet, in einer alkoholischen Lösung mit einer äquimolaren

Menge des entsprechenden Amins versetzt und das Lösungsmittel eindampft.

Verbindungen der Formel I, worin Z eine Carboxy- oder Carboxylatgruppe ($R^1$ Wasserstoff oder ein Kation) bedeutet und $R^2$ die zur Formel I angegebenen Bedeutungen hat, lassen sich ebenfalls verestern. So kann man z.B. ein 11-Deoxy-Hetero-Imino-Prostacyclinderivat der Formel I ($R^1$ = H) bei Temperaturen zwischen -40 und +20°C mit Diazoalkanen der Formel $R^1 - N_1$ ($R^1$ = Alkyl) verestern, wobei die üblichen Lösungsmittel, wie z.B. Diethylether, Tetrahydrofuran, Chloroform oder nieder-molekulare Alkohole wie Methanol verwendet werden können. Die resultierenden Ester können in einfacher Weise durch Eindampfen des Lösungsmittels isoliert und gegebenenfalls chromatographisch gereinigt werden. Eine bevorzugte Veresterungsmethode besteht darin, daß man das Salz des entsprechenden 11-Deoxy-Hetero-Imino-Prostacyclinderivates der Formel I ($R^1$ = Kation) in Gegenwart einer Base, wie z.B. eines Metallalkoholats oder Metallcarbonats in einem geeigneten Lösungsmittel mit einem Alkylierungsmittel der Formel $R^1$-Z', worin $R^1$ die zur Formel I genannten Bedeutungen außer Wasserstoff oder Kation hat, und Z' Halogen, vorzugsweise Brom oder Jod oder den Sulfonsäurerest bedeutet, umsetzt. Als Metallalkoholate kommen z.B. Natriummethylat, Natriumethylat oder Kaliumtertiärbutylat in Betracht, als Carbonate eignen sich z.B. Calciumcarbonat oder Natriumhydrogencarbonat. Als geeignetes Lösungsmittel kommen Alkohole wie z.B. Methanol oder tert. Butanol, Ether wie Tetrahydrofuran oder 1,2-Dimethoxyethan und insbesondere dipolare aprotische Lösungsmittel wie Dimethylformamid, Dimethylsulfoxid, Acetonitril, oder N-Methylpyrrolidon in Betracht.

Die Verbindungen der Formel XIII können als Diastereomerengemisch bezüglich der Stellung der Hydroxylgruppen am Kohlenstoffatom 15 (Prostaglandinnomenklatur), als reine α- oder ß-Isomere oder in Form von optisch aktiven Antipoden für die nachfolgenden Reaktionen eingesetzt werden. Die

Trennung von Stereoisomeren bzw. Antipoden kann aber auch nach jeder folgenden Reaktionsstufe erfolgen. Das bedeutet, daß alle beschriebenen Reaktionen mit Diastereomerengemischen, reinen Diastereomeren oder optisch aktiven Antipoden durchgeführt werden können. Die beanspruchten Verbindungen der Formel I umfassen daher Diastereomerengemische, reine Diastereomere, Epimerengemische und reine Epimeren.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels z.B. Säulen-, Dünnschicht- oder Hochdruckflüssigkeitschromatographie.

Analog den in den Beispielen beschrieben Verbindungen lassen sich nach den erfindungsgemäßen Verfahren auch noch folgende Verbindungen herstellen:

2-Aza-3-(1-thia-4-carboxymethylbutyl-1)-6-(3-(R,S)-hydroxy-6-oxa-1-octenyl)-bicyclo/3,3,0/octen-2

2-Aza-3-(1-thia-4-carboxymethylbutyl-1)-6-(3-(R,S)-hydroxy-4,4-dimethyl-1-octenyl)-bicyclo/3,3,0/octen-2

2-Aza-3-(1-thia-4-carboxymethylbutyl-1)-6-(3-(R,S)-hydroxy-3-phenoxymethyl-1-propenyl)-bicyclo/3,3,0/octen-2

2-Aza-3-(1-thia-4-carboxymethylbutyl-1)-6-(3-(R,S)-hydroxy-4,4-dimethyl-6-oxa-octenyl-1-bicyclo/3,3,0/octen-2

2-Aza-3-(1-thia-4-carboxymethylbutyl-1)-6-(3-(R,S)-hydroxy-3-thienyloxymethyl-1-propenyl)-bicyclo/3,3,0/octen-2

2-Aza-3-(1-thia-4-carboxymethylbutyl-1)-6-(3-(R,S)-hydroxy-4-fluoroctenyl-1-)bicyclo/3,3,0/octen-2

2-Aza-3-(1-thia-4-carboxymethylbutyl-1)-6-(3-(R,S)-hydroxy-3-cyclopentyl-1-propenyl)-bicyclo/3,3,0/octen-2

2-Aza-3-(1-thia-4-carboxymethylbutyl-1)-6-(3-(R,S)-hydroxy-3-thienyl-3-ethyl-1-propenyl)-bicyclo/3,3,0/octen-2

2-Aza-3-(1-thia-4-carboxymethylbutyl-1)-6-(3-(R,S)-hydroxy-3-m-chlorphenoxymethyl-1-propenyl)-bicyclo/3,3,0/octen-2

2-Aza-3-(1-thia-4-carboxymethylbutyl-1)-6-(3-(R,S)-hydroxy-3-(2,2,3,3-tetrafluorcyclobutylethyl-1-propenyl)-bicyclo-/3,3,0/octen-2

2-Aza-3-(1-thia-5-carboxymethylpentyl-1)-6-(3-(R,S)-hydroxy-octenyl-1)-bicyclo/3,3,0/octen-2

2-Aza-3-(1-thia-5-carboxymethylpentyl-1)-6-(3-(R,S)-hydroxy-6-oxy-1-octenyl)-bicyclo/3,3,0/octen-2

2-Aza-3-(1-thia-5-carboxymethylpentyl-1)-6-(3-(R,S)-hydroxy-4,4-dimethyl-1-octenyl)-bicyclo/3,3,0/octen-2

2-Aza-3-(1-thia-5-carboxymethylpentyl-1)-6-(3-(R,S)-hydroxy-3-phenoxymethyl-1-propenyl)-bicyclo/3,3,0/octen-2

2-Aza-3-(1-thia-5-carboxymethylpentyl-1)-6-(3-(R,S)-hydroxy-4,4-dimethyl-6-oxa-octenyl-1)-bicyclo/3,3,0/octen-2

2-Aza-3-(1-thia-5-carboxymethylpentyl-1)-6-(3-(R,S)-hydroxy-3-thienyloxymethyl-1-propenyl)-bicyclo/3,3,0/octen-2

2-Aza-3-(1-thia-5-carboxymethylpentyl-1)-6-(3-(R,S)-hydroxy-4-fluor-octenyl-1)-bicyclo/3,3,0/octen-2

2-Aza-3-(1-thia-5-carboxymethylpentyl-1)-6-(3-(R,S)-hydroxy-3-cyclopentyl-1-propenyl)-bicyclo/3,3,0/octen-2

2-Aza-3-(1-thia-5-carboxymethylpentyl-1)-6-(3-(R,S)-hydroxy-3-thienyl-3-ethyl-1-propenyl)-bicyclo/3,3,0/octen-2

2-Aza-3-(1-thia-5-carboxymethylpentyl-1)-6-(3-(R,S)-hydroxy-3-m-chlorphenoxymethyl-1-propenyl)-bicyclo/3,3,0/octen-2

2-Aza-3-(1-thia-5-carboxymethylbutyl-1)-6-(3-(R,S)-hydroxy-3-(2,2,3,3-tetrafluorcyclobutylethyl)-1-propenyl)-bicyclo-/3,3,0/octen-2

2-Aza-3-(1-thia-4-carboxymethylbuten-3-yl-1)-6-(3-(R,S)-hydroxy-1-octenyl)-bicyclo/3,3,0/octen-2

2-Aza-3-(1-thia-4-carboxymethylbuten-3-yl-1)-6-(3-(R,S)-hydroxy-6-oxa-1-octenyl)-bicyclo/3,3,0/octen-2

2-Aza-3-(1-thia-4-carboxymethylbuten-3-yl-1)-6-(3-(R,S)-hydroxy-4,4-dimethyl-1-octenyl)-bicyclo/3,3,0/octen-2

2-Aza-3-(1-thia-4-carboxymethylbuten-3-yl-1)-6-(3-(R,S)-hydroxy-3-phenoxymethyl-1-propenyl)-bicyclo/3,3,0/octen-2

2-Aza-3-(1-thia-4-carboxymethylbuten-3-yl-1)-6-(3-(R,S)-hydroxy-4,4-dimethyl-6-oxa-octenyl-1)-bicyclo/3,3,0/octen-2

2-Aza-3-(1-thia-4-carboxymethylbuten-3-yl-1)-6-(3-(R,S)-hydroxy-3-thienyloxymethyl-1-propenyl)-bicyclo/3,3,0/octen-2

2-Aza-3-(1-thia-4-carboxymethylbuten-3-yl-1)-6-(3-(R,S)-hydroxy-4-fluoroctenyl-1)-bicyclo/3,3,0/octen-2

2-Aza-3-(1-thia-4-carboxymethylbuten-3-yl-1)-6-(3-(R,S)-hydroxy-3-cyclopentyl-1-propenyl)-bicyclo/3,3,0/octen-2

2-Aza-3-(1-thia-4-carboxymethylbuten-3-yl-1)-6-(3-(R,S)-hydroxy-3-thienyl-3-ethyl-1-propenyl)-bicyclo/3,3,0/octen-2

2-Aza-3-(1-thia-4-carboxymethylbuten-3-yl-1)-6-(3-(R,S)-hydroxy-3-m-chlorphenoxymethyl-1-propenyl)-bicyclo/3,3,0/-octen-2

2-Aza-3-(1-thia-4-carboxymethylbuten-3-yl-1)-6-(3-(R,S)-hydroxy-3-(2,2,3,3-tetrafluorcyclobutylethyl)-1-propenyl)-bicyclo/3,3,0/octen-2

2-Aza-3-(1-aza-4-carboxybutyl-1)-6-(3-(R,S)-hydroxy-6-oxa-1-octenyl)-bicyclo/3,3,0/octen-2

2-Aza-3-(1-aza-4-carboxybutyl-1)-6-(3-(R,S)-hydroxy-4,4-dimethyl-1-octenyl)-bicyclo/3,3,0/octen-2

2-Aza-3-(1-aza-4-carboxybutyl-1)-6-(3-(R,S)-hydroxy-3-phenoxymethyl-1-propenyl)-bicyclo/3,3,0/octen-2

2-Aza-3-(1-aza-4-carboxybutyl-1)-6-(3-(R,S)-hydroxy-4,4-dimethyl-6-oxa-octenyl-1)-bicyclo/3,3,0/octen-2

2-Aza-3-(1-aza-4-carboxybutyl-1)-6-(3-(R,S)-hydroxy-3-thienyloxymethyl-1-propenyl)-bicyclo/3,3,0/octen-2

2-Aza-3-(1-aza-4-carboxybutyl-1)-6-(3-(R,S)-hydroxy-4-fluoroctenyl-1)-bicyclo/3,3,0/octen-2

2-Aza-3-(1-aza-4-carboxybutyl-1)-6-(3-(R,S)-hydroxy-3-cyclopentyl-1-propenyl)-bicyclo/3,3,0/octen-2

2-Aza-3-(1-aza-4-carboxybutyl-1)-6-(3-(R,S)-hydroxy-3-thienyl-3-ethyl-1-propenyl)-bicyclo/3,3,0/octen-2

2-Aza-3-(1-aza-4-carboxybutyl-1)-6-(3-(R,S)-hydroxy-3-m-chlorphenoxymethyl-1-propenyl)-bicyclo/3,3,0/octen-2

2-Aza-3-(1-aza-4-carboxybutyl-1)-6-(3-(R,S)-hydroxy-3-(2,2,3,3-tetrafluorcyclobutylethyl-1-propenyl)-bicyclo-/3,3,0/octen-2

2-Aza-3-(4-carboxymethylanilino)-6-(3-(R,S)-hydroxy-1-octenyl)-bicyclo/3,3,0/octen-2

2-Aza-3-(4-carboxymethylanilino)-6-(3-(R,S)-hydroxy-3-cyclohexyl-1-propenyl)-bicyclo/3,3,0/octen-2

2-Aza-3-(4-carboxymethylanilino)-6-(3-(R,S)-hydroxy-6-oxa-1-octenyl)-bicyclo/3,3,0/octen-2

2-Aza-3-(4-carboxymethylanilino)-6-(3-(R,S)-hydroxy-4,4-dimethyl-1-octenyl)-bicyclo/3,3,0/octen-2

2-Aza-3-(4-carboxymethylanilino)-6-(3-(R,S)-hydroxy-3-phenoxymethyl-1-propenyl)-bicyclo/3,3,0/octen-2

2-Aza-3-(4-carboxymethylanilino)-6-(3-(R,S)-hydroxy-3-(2,2,-3,3-tetrafluorcyclobutylethyl)-1-propenyl)-bicyclo/3,3,0/-octen-2

2-Aza-3-(4-carboxymethylanilino)-6-(3-(R,S)-hydroxy-3-(2-chlor-3-thienyoxymethyl)-1-propenyl)-bicyclo/3,3,0/octen-2

2-Aza-3-(1-oxa-4-carboxymethylbutyl-1)-6-(3-(R,S)-hydroxy-3-thienyloxymethyl-1-propenyl)-bicyclo/3,3,0/octen-2

2-Aza-3-(1-oxa-4-carboxymethylbutyl-1)-6-(3-(R,S)-hydroxy-4,4-dimethyl-1-octenyl)-bicyclo/3,3,0/octen-2

2-Aza-3-(1-oxa-4-carboxymethylbutyl-1)-6-(3-(R,S)-hydroxy-3-phenoxymethyl-1-propenyl)-bicyclo/3,3,0/octen-2

2-Aza-3-(1-oxa-4-carboxymethylpentyl-1)-6-(3-(R,S)-hydroxy-3-thienyloxymethyl-1-propenyl)-bicyclo/3,3,0/octen-2

2-Aza-3-(1-oxa-4-carboxymethylbutyl-1)-6-(3-(R,S)-hydroxy-4,4-dimethyl-1-octenyl)-bicyclo/3,3,0/octen-2

2-Aza-3-(1-oxa-4-carboxymethylbutyl-1)-6-(3-(R,S)-hydroxy-3-phenoxymethyl-1-propenyl)-bicyclo/3,3,0/octen-2

2-Aza-3-( 4-hydroxybutylamino-1)-6-(3-(R,S)-hydroxy-octenyl-1)-bicyclo/3,3,0/octen-2

Die erfindungsgemäßen Produkte der Formel I sind physiologisch sehr wirksam.

So sind diese Verbindungen z.B. wirksam bei der Inhibierung der Blutplättchen-Aggregation, der Verminderung der Hafteigenschaften der Blutplättchen und der Beseitigung oder Verhütung von Thromben bei Säugetieren, einschließlich Menschen. Die Substanzen können daher zur Behandlung und Verhütung von Myocard-Infarkten, zur Behandlung und Verhütung postoperativer Thromben, zum Offenhalten von implantierten Gefäßen und zur Behandlung von Krankheitszuständen wie Atherosklerose, Blutgerinnung durch Lipämie und anderen klinischen Zuständen, bei welchen die zu Grunde liegende Äthiologie mit einem Lipidungleichgewicht oder Hyperlipidämie verbunden sind, verwendet werden. Weitere Anwendungen in vivo sind bei geriatrischen Patienten die Verhütung von zentralen ischiämischen Ausfällen und die Langzeit-Prophylaxe nach Myocard-Infarkten und Schlaganfällen. Die Verbindungen werden zu diesem Zweck systematisch verabreicht, z.B. intravenös, oral, subkutan, intramuskulär oder in Form steriler Implantate zur verlängerten Wirkung. Zur raschen Wirkung wird die intravenöse Verabreichung bevorzugt. Man verwendet Dosen von etwa 0,15 bis 150 µg/kg Körpergewicht pro Tag, insbesondere 0,5 bis 1,0 µg/kg, wobei die genaue Menge von Alter, Gewicht und Zustand der Patienten oder des Tieres, der Häufigkeit und Art der Verabreichung abhängt.

Beim Zusatz dieser Verbindungen zu Gesamtblut gelangt man zu Anwendungen in vitro, wie z.B. bei der Lagerung von Gesamtblut zur Verwendung in Herz/Lungen-Maschinen. Das diese Verbindungen enthaltende Blut kann auch durch Organe, z.B. Herz und Nieren, zirkulieren, die einem Spender entnommen wurden und zur Verpflanzung bereitstehen. Die erfindungsgemäßen Verbindungen sind auch brauchbar zur Herstellung von an Blutplättchen reichen Konzentraten zur Verwendung bei Thrombocytopenie, Chemotherapie und Bestrahlungstherapie.

Bei Anwendungen in vitro verwendet man 0,01 bis 1,0 µg/ml Gesamtblut.

Insbesondere sind Verbindungen der Formel I auch brauchbar als hypotensive Mittel zur Herabsetzung des Blutdrucks bei Säugetieren einschließlich Menschen. Zu diesem Zweck erfolgt die Verabreichung oral in Dosen von etwa 1,5 µg bis 1,5 mg/kg Körpergewicht, bevorzugt 5 µg bis 1 mg/kg Körpergewicht pro Tag, in Einzeldosen von 50 µg bis 20 mg pro Patient oder durch intravenöse Infusion in einer Menge von etwa 0,001 bis etwa 1 µg/kg Körpergewicht pro Minute, oder mehreren Dosen von etwa 1,5 bis 150 µg/kg Körpergewicht pro Tag.

Die Prostaglandinderivate sind auch brauchbar bei Säugetieren einschließlich Menschen sowie bestimmten Nutztieren, z.B. Hunden und Schweinen, zur Verminderung und Steuerung übermäßiger Magensaftsekretion, womit die Bildung von Magen-/Darmgeschwüren vermindert oder vermieden werden und die Heilung solcher bereits vorhandener Geschwüre beschleunigt werden kann. Zu diesem Zweck werden die Verbindungen intravenös, subkutan oder intramuskulär injiziert oder infundiert. Das Dosierungsschema für das Prostaglandin hängt bei dieser Behandlung von verschiedenen Faktoren ab einschließlich Typ, Alter, Gewicht, Geschlecht und medizinischem Zustand des Patienten, dem Dosierungsschema des entzündungshemmenden Synthetase-Inhibitors und der Empfindlichkeit des Patienten auf den Synthetase-Inhibitor bezüglich der Magen-/Darmwirkung. So empfindet z.B. nicht jeder Patient, der eine entzündungshemmende Substanz benötigt, die gleichen unangenehmen gastrointestinalen Effekte. Diese ändern sich vielmehr häufig in Art und Ausmaß. Es liegt daher im Erfahrungsbereich des Arztes oder Tierarztes, festzustellen, ob die Verabreichung der entzündungshemmenden Substanz unerwünschte gastrointestinale Effekte beim Mensch oder Tier erzeugt und die wirksame Menge des Prostaglandins zu verschreiben, mit der diese Effekte im wesentlichen eliminiert werden können.

Einzelne Vertreter dieser Substanzen eignen sich zur Behandlung von Asthma. Sie sind beispielsweise nützlich als Bronchiendilatoren oder als Inhibitoren von Mediatoren, wie z.B. SRS-A und Histamin, die aus durch einen Antigen-/Antikörper-Komplex aktivierten Zellen freigesetzt werden. Die Verbindungen bekämpfen daher Krämpfe und erleichtern das Atmen bei Krankheitszuständen wie Bronchialasthma, Bronchitis, Bronchiectase, Pneumonie und Emphysem. Für diese Zwecke werden die Verbindungen in verschiedenen Dosierungsformen verabreicht, z.B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

 0055851

Beispiel 1:

(1-Benzyloxymethyl-3-hydroxycyclopentyl-2)essigsäure-amid

122.5 g Lacton werden in 0.5 l Methanol mit 150 ml flüssigem Ammoniak und 0.2 - 0.4 mol % 2-Pyridon 36 Stunden im Schüttelautoklaven bei 130°C behandelt. Das Lösungsmittel wird abgezogen, und der Rückstand aus Aceton kristallisiert. Die Mutterlauge kann an Kieselgel (Laufmittel: Essigester) filtriert werden.

Ausbeute: 90 g (68.7 %)

Schmp.: 123 - 124°C

NMR (DMSO): δ ppm: 7.3, s, 5H (Aryl-H); 7.1, 6.7, s (breit), 2H (NH); 4.2, s, 2H ($CH_2$ - Ø); 4.0, m, 1H (CH - OH); 3.3, m, 2H ($CH_2$ - O);

IR (KBr): $cm^{-1}$: 3400 (breit) NH, OH);
1670 (C = O Amid);
1600 (Aromat);

RF (Essigester/ Methanol 8:1): 0.26

Beispiel 2:

(3-Benzyloxymethylcyclopentanon-1-yl-2)essigsäureamid

Zu einer Lösung von 48 g Hydroxyamid (0.182 mol) (Beispiel 1) werden bei -20°C unter Rühren langsam 100 ml JONES-Lösung (0.25 mol) getropft. Danach wird noch 2 Stunden bei dieser Temperatur gerührt. Der Überschuß an Oxidationsmittel wird durch Zugabe von Isopropanol beseitigt. Die grüne Suspension wird mit 100 g $MgSO_4$ versetzt und mit Triethylamin neutralisiert. Man läßt den Ansatz auf Raumtemperatur kommen und saugt über ein Celite-Filter ab. Das Filtrat wird eingeengt und der Rückstand in Essigester aufgenommen, mit Wasser versetzt und die Phasen getrennt. Die wässrige Phase wird mit Kochsalz gesättigt und mit Essigester zurückextrahiert. Die vereinigten Extrakte werden getrocknet und eingeengt. Der Rückstand wird über eine kurze Säule mit Kieselgel (Laufmittel: Essigester/ Methanol 10:1) filtriert.

Ausbeute: 19 g (61 %)

Im DC (Essigester/ Methanol 8:1) ist zunächst ein schneller Fleck mit RF = 0.40 zu beobachten (=Ketoamid). Beim Stehenlassen taucht ein Fleck mit Rf = 0.27 auf, der mit der Zeit immer stärker wird (=Hydroxylactam).

NMR ($CDCl_3$)

Ketoamid: δ ppm: 7.3, s, 5H (Aryl-H); 5.9, s (breit), 2H (NH; 4.55, s, 2H ($CH_2$ - Ø); 3.6, d, 2H ($CH_2$-O);

Hydroxylactam: δ ppm: 7.3, s, 5H (Aryl-H); 6.9, s (breit), 1H (NH); 4.5, s, 2H ($CH_2$-Ø); 3.7 - 4.5, m (breit), 1H (OH; 3.45, d, 2H ($CH_2$-O);

IR (Film): $cm^{-1}$:

| | |
|---|---|
| Ketoamid: | 3200, 3320 (NH), 1735 (C=O Fünfring), 1665 (C=O Amid); |
| Hydroxylactam: | 3300 (breit) (OH), 1680 (C=O Amid); |

Beispiel 3:

1- Phenylthio-2-aza-3-oxo-6-benzyloxymethylbicyclo [3.3.0] octan

Zu einer Lösung von 29 g Ketoamid/ Hydroxylactam (0.111 mol) (Beispiel 2) in 280 ml $CH_2Cl_2$/ Pyridin abs. 1:1 werden bei Raumtemperatur 12.87 g Thiophenol (= 12 ml, 0.117 mol) getropft. Danach werden 19.5 g Trimethylchlorsilan (= 22.75 ml, 0.179 mol) langsam zugetropft, und der Ansatz auf 70°C Außentemperatur hochgeheizt. Der Ansatz wird über Nacht bei dieser Temperatur gehalten. Danach läßt man abkühlen, saugt den Niederschlag ab und engt das Filtrat ein. Der Rückstand wird in Essigester aufgenommen und mit halbges. NaCl gesättigt und mit Essigester extrahiert. Die vereinigten Extrakte werden über $MgSo_4$ getrocknet und eingeengt. Der Rückstand kann ohne weitere Reinigung weiterverarbeitet werden.

Ausbeute: 46 g ( >100 %).

Schmp.: 65 - 68°C

| | |
|---|---|
| NMR ($CDCL_3$): δ ppm: | 7.2 - 7.65, m, 10H (Aryl-H); 4.5, s, 2H (O-$CH_2$-Aryl); 3.4, d, 2H ($CH_2$-O); |
| IR (Film): $cm^{-1}$: | 3200 (breit) (NH); 3060 (Aryl-H); 1690 (C=O Lactam) |

RF (Essigester/ Methanol 8:1) : 0.59 .

Beispiel 4:

2-Aza-3-oxo-6-benzyloxymethylbicyclo [3.3.0] octan

43 g Thioether (0.12 mol) (Beispiel 3) werden mit ca. 250 g RANEY-Nickel (wasserfrei) in 700 ml t-Butanol 30 min. auf 50°C erwärmt. Der Katalysator wird unter Schutzgas über ein Celite-Filter abgesaugt, gründlich mit Methanol nachgewaschen, und das Filtrat eingeengt. Der Rückstand wird über eine kurze Säule mit Kieselgel (Laufmittel: Essigester) filtriert.

Ausbeute: 19.4 g (65 %)

NMR ($CDCl_3$): δ ppm: 7.2, s, 5H (Aryl-H); 7.0, s (breit), 1H (NH); 4.45, s, 2H (O-$CH_2$-Aryl); 3,8 - 4.2, m, 1H (O=C-N-CH); 3.35, d, 2H ($CH_2$-O)

IR (Film): $cm^{-1}$: 3200 (breit, NH); 3020 - 3080 (Aryl-H); 1690 (C=O Lactam)

Rf (Essigester/ Methanol 8:1) : 0.34

Beispiel 5:

2-Aza-3-oxo-6-hydroxymethylbicyclo [3.3.0] octan

19.4 g Benzylether (Beispiel 4) werden in 700 ml Methanol mit 10 g Palladium auf Kohle 10 % unter Zusatz von 10 ml Eisessig bei 50°C und Normaldruck hydriert. Der Katalysator wird über ein Celite-Filter abgesaugt, gut mit Methanol nachgewaschen und das Filtrat mit $NaHCO_3$-Lösung neutralisiert. Nach Einengen zur Trockene wird mit THF ausgekocht. Nach Abziehen des Lösungsmittels verbleibt ein zähes Öl.

Ausbeute: 11.4 g (93 %)

NMR (DMSO): $\delta$ ppm: 7.5, s (breit), 1H (NH); 4.55, m, 1H (OH); 3.95, m 1H (O=C-N-C-H); 3.3, m, 2H ($CH_2$-O)

IR (Film): $cm^{-1}$: 3300 (breit, NH, OH); 1670 (C=O Lactam)

Rf (Essigester/ Methanol 8:1): 0.14

Beispiel 6:

2-Aza-3-oxo-6-(3-oxo-1-octenyl)bicyclo [3.3.0] octan

Eine Lösung von 1.63 g DMSO (20.9 mmol, 1.48 ml) in 25 ml absolutem Methylenchlorid wird unter Argon auf -60°C abgekühlt. 1.2 g Oxalylchlorid (9.6 mmol, 0.84 ml frisch dest.!) werden zugetropft und noch 10 min. gerührt.

Danach wird eine Lösung von 1.0 g Alkohol (8.7 mmol) (Beispiel 5) in 15 ml Methylenchlorid/ 1.5 ml DMSO zugetropft und noch 20 min bei -60°C gerührt. Bei dieser Temperatur werden 4.4 g Triethylamin (43.5 mmol, 6.06 ml) zugegeben, und die Lösung noch 30 min nachgerührt. Danach wird die Lösung durch Zugabe von ethanolischer HCl-Lösung oder durch Einleiten von HCl-Gas neutralisiert und auf Raumtemperatur kommen lassen. Nach Einengen wird der Rückstand in Essigester aufgenommen, das ausgefallene Ammoniumsalz abfiltriert, mit Essigester gewaschen und das Filtrat erneut eingeengt. Der Rückstand wird nach Trocknen am Hochvakuum direkt weiterverwendet. Zu einer Suspension von 380 mg 55 % NaH-Dispersion (209 mg NaH, 8.7 mmol) in 150 ml trockenem DME werden 1.93 g 2-Oxoheptylphosponat (8.7 mmol, 1.81 ml) getropft und bei Raumtemperatur so lange gerührt, bis die Wasserstoffentwicklung beendet ist. Danach wird die Lösung des rohen Aldehyds in DME zugeben und über Nacht bei Raumtemperatur stehen lassen. Nach Neutralisation mit Essigsäure wird eingeengt, der Rückstand in Essigester aufgenommen und mit halbgesättigter NaCl-Lösung gewaschen. Die wässrige Phase wird mit NaCl gesättigt und mit Essigester extrahiert. Die vereinigten organischen Phasen werden über $MgSO_4$ getrocknet und eingeengt. Der Rückstand wird mit Ether/ Petrolether angerieben und die Kristalle abgesaugt.

Ausbeute: 950 mg (59 % bezogen auf Alkohol)

Schmp.: 99 - 100°C

NMR ($CDCl_3$): $\delta$ ppm: 5.9 - 6.9, m, 3H (H-C=C-H, NH);
3.95 - 4.3, m, 1H (O=C-N-C-H)

IR (KBr): $cm^{-1}$: 3200 (breit, NH); 3080 (H-C=C); 1685 (C=O Lactam); 1635 (C=C)

Rf Aldehyd (Essigester/ Methanol 4:1): 0.33

Rf Enon (Essigester/ Methanol 4:1) : 0.41

Beispiel 7:

2-Aza-3-oxo-6-(3-(R,S)-hydroxy-1-octenyl)bicyclo [3.3.0] octan

2.04 g Enon (8.18 mmol) (Beispiel 6) werden mit 30 ml einer Toluol-Lösung von Di-isobornyl-Aluminium-isopropylat (0.44 mol/l; 13.2 mmol) in 50 ml trockenem Toluol über Nacht bei Raumtemperatur gerührt. Die Lösung wird mit gesättigter Na/K-Tartrat-Lösung auf pH 5 gestellt, die organische Phase abgetrennt, die wässrige Phase mit NaCl gesättigt und mehrmals mit Essigester extrahiert. Die vereinigten organischen Phasen werden über $MgSO_4$ getrocknet und eingeengt. Der Rückstand wird mit Pentan angerieben, die ausgefallenen Kristalle werden abgesaugt.

Ausbeute: 1.77 g (86.1 %)

Schmp.: 90°C

NMR ($CDCl_3$): δ ppm: 6.1, s (breit), 1H (NH); 5.4 - 5.6, m, 2H (H-C = C-H); 3.9 - 4.3, m, 2H (O=C-N-C-H, CH-O)

IR (KBr): $cm^{-1}$: 3500 - 3000 (OH, NH); 1685 (C=O Lactam)

Rf ($CH_2Cl_2$/ Methanol 20:1): 0. 127 (β-Isomeres), 0.093 (α -Isomeres)

0055851

Beispiel 8:

2-Aza-3-oxo-6-(3-(R,S)-acetoxy-1-octenyl)bicyclo [3.3.0]octan

740 mg Alkohol (2.94 mmol) (Beispiel 7) werden mit 974 mg frisch destilliertem Acetanhydrid (0.9 ml, 9.54 mmol) in 10 ml trockenem Pyridin über Nacht bei Raumtemperatur gerührt. Die Lösung wird eingeengt, und der Rückstand in Essigester aufgenommen, mit Wasser gewaschen und getrocknet. Nach Einengen verbleibt ein farbloses Öl.

Ausbeute: 750 mg (68.8 %)

NMR ($CDCl_3$): $\delta$ ppm: 5.9 - 6.4, s (breit), 1H (NH);
5.4 - 5.6, m, 2H (H-C = C-H);
4.95 - 5.35, m, 1H (H-C-OAc);
3.9 - 4.25, m 1H (O = C-N-C-H);
2.0, s, 3H (O = C-$CH_3$)

IR (Film): $cm^{-1}$: 3200 (breit, NH); 1735 (C = O Ester);
1690 (C = O Lactam)

Rf (Essigester/ Methanol 8:1) : 0.41

Beispiel 9:

2-Aza-3-thio-6-(3-(R,S)-acetoxy)bicyclo [3.3.0]octan

420 mg Lactam (1.43 mmol) (Beispiel 8) werden mit 1.42g $P_4S_{10}$x 4Py-Komplex (1.86 mmol) in 10 ml Pyridin/ Toluol 1:1 4 Stunden bei 80°C gerührt. Die gelbe Lösung wird abgesaugt, und der Rückstand mehrfach mit Toluol ausgekocht. Die so erhaltene Lösung wird eingeengt, mit Essigester aufgenommen und mit Wasser gewaschen. Die wässrige Phase wird mit NaCl gesättigt und mit Essigester zurückextrahiert. Die vereinigten organischen Phasen werden über $MgSO_4$ getrocknet und eingeengt.

Ausbeute: 360 mg (81.3 %)

NMR ($CDCl_3$): δ ppm: 7.6 - 8.0, s (breit, NH);
5.35 - 5.55, m, 2H (H-C = C-H);
4.95 - 5.35, m, 1H (H-C-OAc);
4.15 - 4.5, m, 1H (S = C-N-C-H);
2.0, s, 3H (O = C-$CH_3$)

IR (Film): $cm^{-1}$: 1725 (C=O Ester);
1525 (C=N Thiolactam)

Rf (Essigester/ Methanol 8:1) : 0.57

Beispiel 10:

2-Aza-3-(1-thia-4-carbethoxybutyl-1)-6-(3-(R,S)-acetoxy)bicyclo [3.3.0]octen-2

Zu einer Suspension von 74 mg 55 % NaH-Dispersion (40.8 mg NaH 1.7 mmol) in 5 ml trockenem DME werden bei Raumtemperatur 510 mg Thiolactam (1.65 mmol) (Beispiel 9) in 1 ml DME getropft und so lange nachgerührt, bis die Wasserstoffentwicklung beendet ist. Danach werden 341.4 mg 4-Brombuttersäureethylester (1.75 mmol, 0.25ml) zugegeben und noch 4 Stunden gerührt. Die erhaltene Suspension wird eingeengt, mit Essigester aufgenommen und mit Wasser gewaschen. Die wässrige Phase wird mit NaCl gesättigt und mit Essigester extrahiert. Die vereinigten Extrakte werden über $MgSO_4$ getrocknet und eingeengt.

Ausbeute: 410 mg (58.7 %)

NMR ($CDCl_3$): $\delta$ ppm: 5.35 - 5.6, m, 2H (H-C = C-H);
5.0 - 5.35, m, 1H (H-C-OAc);
4.3 - 4.7, m, 1H (C=N-C-H);
4.1, q, 2H (O=C-O-$CH_2$);
3.05, t, 2H (S-$CH_2$);
2.05, s, 3H (O=C-$CH_3$);
1.25, t, 3H ($CH_2$-$CH_3$)

IR (Film): $cm^{-1}$: 3030 (H-C=);
1730 (C = O Ester);
1590 (C = N)

Rf (Essigester/ cyclohexan 1:1) : 0.41

Beispiel 11:

2-Aza-3-(1-thia-4 -carbomethoxybutyl-1)bicyclo [3.3.0]octen-2

230 mg Acetat (0.54 mmol) (Beispiel 10) werden mit 37 mg $K_2CO_3$ (0.27 mmol, 5 Stunden bei 80°C am Hochvakuum getrocknet) in 2.3 ml trockenem Methanol so lange gerührt, bis im DC das Ausgangsmaterial verschwunden ist. Danach wird mit 0.54 ml 1n HCl neutralisiert, mit Essigester verdünnt und die organische Phase abgetrennt. Die wässrige Phase wird mit NaCl gesättigt und mit Essigester extrahiert. Die vereinigten organischen Phasen werden über $MgSo_4$ getrocknet und eingeengt. Der Rückstand wird über eine kurze Säule mit Kieselgel (Laufmittel: Essigester) filtriert.

Ausbeute: 133 mg (64.5 %)

NMR ($CDCl_3$): $\delta$ ppm: 5.4 - 5.55, m, 2H (H-C = C-H);
4.25 - 4.7, m, 1H (C=N-C-H);
3.9 - 4.2, m, 1H (CH-O);
3. 65, s, 3H ($CO_2CH_3$);
3.1, t, 2H ($S-CH_2$)

IR (Film): $cm^{-1}$: 3400 (breit, OH);
1740 (C=O Ester);
1590 (C=N)

Rf (Essigester/ Cyclohexan 1:1) : 0.16

Beispiel 12:

2-Aza-3-methylthio-6-(3-(R,S)-acetoxy)bicyclo[3,3,0] octen-2

190 mg Thiolactam (0,614 mmol) (Beispiel 9) werden in 2 ml abs. DME gelöst und zu einer Suspension von 15,6 mg NaH (0.65 mmol, = 28,4 mg 55 % Dispersion) in 3 ml DME getropft.Es wird so lange bei RT gerührt, bis die Wasserstoffentwicklung beendet ist. Danach gibt man 92,3 mg Methyljodid (0.65 mmol, 40.5 µl) zu, und rührt noch 4 - 6 Stunden. Das Lösungsmittel wird abgezogen, der Rückstand in Essigester aufgenommen und mit ges. NaCl-Lösung gewaschen, getrocknet und eingeengt. Der Rückstand kann ohne Reinigung weiter umgesetzt werden.

Ausbeute: 170 mg (85,6 %)
NMR ($CDCl_3$): δ ppm: 5,5 - 5,7, m, 2H (H-C=C-H); 5,05 - 5,35, m, 1H (H-C-OAc), 4,3 - 4,7, m, 1 H (C=N-C-H); 3,1, s, 3 H ($S-CH_3$); 2,05, s, 3 H ($O=C-CH_3$)
Rf (Essigester/Cyclohexan 1:1): 0,35

Beispiel 13:

2-Aza-3-(1-aza-4-carboxybutyl-1)-6-(3-(R,S)-acetoxy) bicyclo[3,3,0]octen-2

170 mg Thiomethylether (0,526 mmol) (Beispiel 12) werden in 2 ml abs. Methanol mit 54,2 mg γ-Aminobuttersäure (0,526 mmol) unter Rückfluß gekocht. Das Lösungsmittel wird abgezogen, und der Rückstand an Kieselgel(Lauf-

0055851

mittel:Essigester/Methanol 4:1) filtriert.

Ausbeute: 123 mg (62 %)

NMR (DMSO): δ ppm: 5,5 - 5,6, m, 2 H (H-C=C-H); 5,55 - 5,7, m, 1 H (H-C-OAc); 4,25 - 4,4, m, 1 H (C=N-C-H); 3,2, t, 2 H ($N-CH_2$); 2,05, s, 3 H ($O=C-CH_3$)

Rf (Essigester/Methanol 4:1): 0,43

Beispiel 14:

2-Aza-3-(1-aza-4-carboxybutyl-1)-6-(3-(R,S)-hydroxy) bicyclo[3,3,0]octen-2

120 mg Acetat (0,317 mmol) (Beispiel 13) werden mit 22 mg $K_2CO_3$ (0,16 mmol) in 1,2 ml absolutem Methanol bei Raumtemperatur gerührt. Nach Neutralisation mit Salzsäure wird eingeengt, und der Rückstand mit THF ausgekocht. Das Lösungsmittel wird abgezogen und der Rückstand an Kieselgel (Laufmittel:Essigester/Methanol 4:1) chromatographiert.

Ausbeute: 52 mg (74 %)

NMR (DMSO): δ ppm: 5,4 - 5,55, m, 2 H (H-C=C-H); 4,2 - 4,35, m, 1 H (N-C-C-H); 3,8 - 3,95, m, 1H (CH-O); 3,15 - 3,3, m, 2 H ($N-CH_2$)

Rf (Essigester/Methanol 4:1) : 0,24

Beispiel 15:

2-Aza-3-(4-dimethylaminobutylamino)-6-(3-(R,S)-acetoxy)bicyclo[3,3,0]octen-2

20 mg Methylthioether (0,372 mmol) (Beispiel 12) werden mit 33,5 mg Dimethylaminobutylamin (0,372 mmol) und 0,5 ml Eisessig in 1,2 ml Methanol unter Rückfluß gekocht. Das Lösungsmittel wird abgezogen, der Rückstand in Essigester aufgenommen und mit 1/2 gesättigter Kochsalzlösung gewaschen, getrocknet und eingeengt. Der Rückstand wird über eine kurze Säule mit Kieselgel (Laufmittel:Essigester/Methanol 4:1) filtriert.

Ausbeute: 99 mg (68 %)
NMR (DMSO): δ ppm: 5,4 - 5,6, m, 2 H (H-C=C-H);
5,1 - 5,3, m, 1 H (H-C-OAc);
4,3 - 4,7, m, 1 H (C=N-C-H);
3,2, t, 2 H ($N-CH_2$); 2,2, s, 6 H ($N(CH_3)_2$) 2,05, s, 3 H ($O=C-CH_3$)

Rf (Essigester/Methanol 4:1) : 0,38

Beispiel 16:

2-Aza-3-(4-dimethylaminobutylamino)-6-(3-(R,S)-hydroxy)bicyclo[3,3,0]octen-2

90 mg Acetat (0,23 mmol) (Beispiel 15) werden mit 22,8 mg $K_2CO_3$ (0,165 mmol) in 1 ml $CH_3OH$ bei Raumtemperatur gerührt. Nach Neutralisation mit Salzsäure wird zur Trockene eingeengt, der Rückstand mit THF ausgekocht und das Lösungsmittel abgezogen. Der Rückstand wird an Kieselgel (Laufmittel:Essigester/Methanol 4:1) chromatographiert.

Ausbeute: 57 mg (71 %)

NMR (DMSO): δ ppm: 5,5 - 5,7, m, 2 H (H-C=C-H); 4,3 - 4,7, m, 1 H (C=N-C-H); 3,7 - 3,9, m, 1 H (CH-O); 3,2, t, 2 H ($N-CH_2$); 2,2, s, 6 H ($N(CH_3)_2$)

Rf (Essigester/Methanol 4:1) : 0,28

Beispiel 17:

2-Aza-3-(1-oxa-4-carbethoxybutyl-1)-6-(3-(R,S)-acetoxy-1-octenyl)bicyclo[3,3,0]octen-2

140 mg Lactam (0,477 mmol) (Beispiel 8) und 195 mg 4-Brombuttersäureethylether (1 mmol) werden in 2 ml trockenem Xylol gelöst. Die Lösung wird auf 60°C erwärmt und mit 139 mg Silberoxid (0,6 mmol) versetzt. und 6 Stunden auf 140 - 160°C erwärmt. Nach Abkühlen wird mit 5 ml Diethylether versetzt, filtritert, und das Filtrat eingeengt. Der Rückstand wird an Kieselgel (Laufmittel:Cyclohexan/Essigester 1:1) chromatographiert.

Ausbeute: 128 mg (66 %)

NMR ($CDCl_3$): δ ppm: 5,5 - 5,7, m, 2 H (H-C=C-H); 5,1 - 5,4, m, 1 H (H-C-OAc); 4,1 - 4,3, m, 1 H (C=N-C-H); 4,1, q, 2 H ($CO_2-CH_2$); 4,15, t, 2 H ($N=C-O-CH_2$); 2,05, s, 3 H

(O = C-$CH_3$); 1,25, t, 3 H ($CH_2$-$CH_3$)

Rf (Essigester/Cyclohexan 1:1) : 0,39

Beispiel 18:

2-Aza-3-(1-oxa-4-carbomethoxybutyl-1)-6-(3-(R,S)-hydroxy-1-octenyl)bicyclo[3,3,0]octen-2

120 mg Acetat (0,295 mmol) (Beispiel 17) werden mit 20,4 mg $K_2CO_3$ (0,148 mmol) in 1,2 ml absolutem Methanol bei Raumtemperatur gerührt. Nach Neutralisation mit Salzsäure wird die Lösung mit Essigester versetzt und mit NaCl-Lösung gewaschen, getrocknet und eingeengt. Der Rückstand wird an Kieselgel (Laufmittel: Essigester/Cyclohexan 1:1) chromatographiert.

Ausbeute: 73 mg (68 %)

NMR ($CDCl_3$): $\delta$ ppm: 5,5 - 5,7, m, 2 H (H-C=C-H); 4,1 - 4,3, m, 1 H (C=N-C-H); 4,15, t, 2 H (N=C-O-$CH_2$); 3,8 - 4,0, m, 1 H (CH-O); 3,7, s, 3 H ($CO_2CH_3$)

Rf (Essigester/Cyclohexan 1:1) : 0,12

Patentansprüche:

1. Verbindungen der Formel I

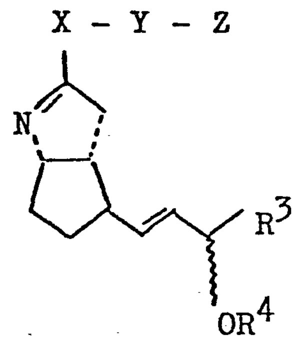

(I)

in welcher bedeuten

X ein Sauerstoff- oder Schwefelatom oder eine NH-Gruppe;

Y einen geradkettigen oder verzweigten Alkylenrest mit bis zu 8 Kohlenstoffatomen oder einen geradkettigen oder verzweigten ungesättigten aliphatischen Rest mit 3 bis 8 Kohlenstoffatomen oder einen cycloaliphatischen Rest mit 3 bis 6 Kohlenstoffatomen oder einen Phenylenrest;

Z einen Rest der Formel $-CO_2R^1$, $-CH_2OH$ oder $CH_2-N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder ein Ammoniumion, das sich von einem primären, sekundären oder tertiären Amin ableitet, oder ein Tetraalkylammonium-Ion,

$R^2$ Wasserstoff oder einen geradkettigen oder verzweigten aliphatischen Kohlenwasserstoffrest mit

bis zu 5 C-Atomen oder $R^2$-$R^2$ zusammen auch eine $-(CH_2)_n$-Gruppe mit n = 3 bis 6

$R^3$ einen Arylrest, der im Kern 1- bis 3-fach substituiert sein kann mit Halogen, Trifluormethyl und/oder Alkyl oder Alkoxy mit je 1 bis 6 C-Atomen, oder einen cycloaliphatischen Rest mit 3 bis 8 Kohlenstoffatomen oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, wobei Alkyl- und aliphatischer Kohlenwasserstoffrest ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Phenyl oder einem α- oder ß-Thienyl- oder α- oder ß-Furylrest, die ihrerseits im Kern 1- bis 3-fach substituiert sein können mit Halogen, Trifluormethyl und/ oder Alkyl oder Alkoxy mit je 1 bis 6 C-Atomen,

c) einem Phenoxy-, einem α- oder ß-Thienyloxyrest oder einem Cycloalkoxyrest mit 3 bis 7 Kohlenstoffatomen, wobei die genannten Reste ihrerseits im Kern 1- bis 3-fach substituiert sein können mit Halogen, Trifluormethyl und/oder Alkyl oder Alkyloxy mit je 1 bis 6 C-Atomen,

$R^4$ Wasserstoff oder eine unter neutralen oder basischen Bedingungen leicht abspaltbare Schutzgruppe.

2. Verfahren zur Herstellung von Verbindungen der Formel I

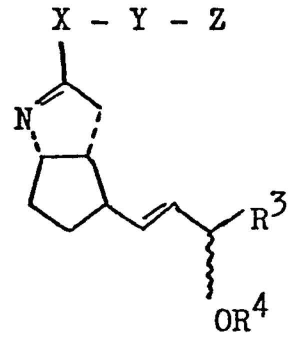

(I)

in welcher bedeuten

X ein Sauerstoff- oder Schwefelatom oder eine NH-Gruppe;

Y einen geradkettigen oder verzweigten Alkylenrest mit bis zu 8 Kohlenstoffatomen oder einen geradkettigen oder verzweigten ungesättigten aliphatischen Rest mit 3 bis 8 Kohlenstoffatomen oder einen cycloaliphatischen Rest mit 3 bis 6 Kohlenstoffatomen oder einen Phenylenrest;

Z einen Rest der Formel $-CO_2R^1$, $-CH_2OH$ oder $CH_2-N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder ein Ammoniumion, das sich von einem primären, sekundären oder tertiären Amin ableitet, oder ein Tetraalkylammonium-Ion,

$R^2$ Wasserstoff oder einen geradkettigen oder verzweigten aliphatischen Kohlenwasserstoffrest mit bis zu 5 C-Atomen oder $R^2$-$R^2$ zusammen auch eine $-(CH_2)_n$-Gruppe mit n = 3 bis 6

$R^3$ einen Arylrest, der im Kern 1- bis 3-fach substituiert sein kann mit Halogen, Trifluormethyl und/

oder Alkyl oder Alkoxy mit je 1 bis 6 C-Atomen, oder einen cycloaliphatischen Rest mit 3 bis 8 Kohlenstoffatomen oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, wobei Alkyl- und aliphatischer Kohlenwasserstoffrest ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Phenyl oder einem α- oder ß-Thienyl- oder α- oder ß-Furylrest, die ihrerseits im Kern 1- bis 3-fach substituiert sein können mit Halogen, Trifluormethyl und/oder Alkyl oder Alkoxy mit je 1 bis 6 C-Atomen,

c) einem Phenoxy-, einem α- oder ß-Thienyloxyrest oder einem Cycloalkoxyrest mit 3 bis 7 Kohlenstoffatomen, wobei die genannten Reste ihrerseits im Kern 1- bis 3-fach substituiert sein können mit Halogen, Trifluormethyl und/oder Alkyl oder Alkyloxy mit je bis 6 C-Atomen,

$R^4$ Wasserstoff oder eine unter neutralen oder basischen Bedingungen leicht abspaltbare Schutzgruppe,

dadurch gekennzeichnet, daß man

a) eine Verbindung der Formel XIV oder XV

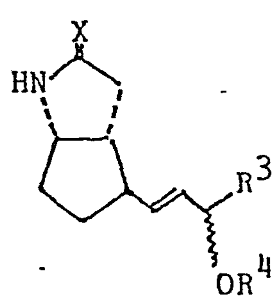

(XIV)
(XV)

worin $R^3$ die oben genannte Bedeutung hat, $R^4$ eine unter neutralen oder basischen Bedingungen leicht abspaltbare Schutzgruppe darstellt und X ein Sauerstoff- oder Schwefelatom bedeutet, mit einem Alkylhalogenid der Formel XVI

$$Z - Y - Hal \qquad (XVI)$$

worin Hal Jod, Chlor oder Brom bedeutet und Y und Z die oben genannten Bedeutungen haben, alkyliert zu einer Verbindung der Formel I, worin X ein Sauerstoff- oder Schwefelatom darstellt, oder

b) eine Verbindung der Formel XVII

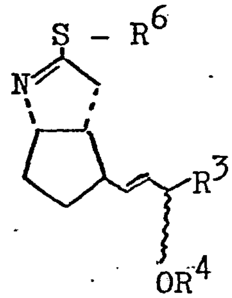

(XVII)

worin $R^3$ und $R^4$ die oben genannten Bedeutungen haben und $R^6$ einen $C_{1-4}$-Alkylrest bedeutet, mit einem Amin der Formel XVIII

$$Z - Y - NH_2 \qquad (XVIII)$$

worin Z und Y die oben genannten Bedeutungen haben, umsetzt zu einer Verbindung der Formel I, worin X den NH-Rest bedeutet,

c) gegebenenfalls in einer Verbindung der Formel I die Schutzgruppe $R^4$ durch basisch katalysierte Hydrolyse abspaltet,

d) gegebenenfalls eine Verbindung der Formel I, in welcher Z den Rest $CO_2R^1$ bedeutet, wobei $R^1$ den oben genannten Rest jedoch nicht Wasserstoff oder ein Kation darstellt und $R^4$ Wasserstoff bedeutet, zu einer Verbindung der Formel I

verseift, in welcher Z den Rest $CO_2R^1$ darstellt, wobei $R^1$ Wasserstoff oder ein physiologisch verträgliches Kation bedeutet,

e) gegebenenfalls eine Verbindung der Formel I, in welcher Z den Rest $CO_2R^1$ darstellt, wobei $R^1$ Wasserstoff oder ein Kation bedeutet, zu einer Verbindung der Formel I verestert, worin Z den Rest $CO_2R^1$ darstellt, wobei $R^1$ die zur Formel I genannten Bedeutungen hat, jedoch nicht Wasserstoff oder ein Kation bedeutet, und

f) gegebenenfalls in einer Verbindung der Formel I, in welcher Z den Rest $CO_2R^1$ darstellt, wobei $R^1$ ein physiologisch verträglicher Kation darstellt, dieses Kation gegen ein anderes austauscht.

3. Verfahren zur Herstellung von Arzneimitteln, dadurch gekennzeichnet, daß man eine Verbindung der Formel I, gegebenenfalls mit üblichen pharmazeutischen Trägern und/oder Stabilisatoren, in eine therapeutisch geeignete Dareichungsform bringt.

4. Arzneimittel, gekennzeichnet durch den Gehalt an einer Verbindung der Formel I in Mischung mit einem pharmazeutisch üblichen Träger und/oder Stabilisator.

5. Verbindungen der Formel I zur Verwendung als Arzneimittel.

Patentansprüche für Österreich:

1. Verfahren zur Herstellung von Verbindungen der Formel I

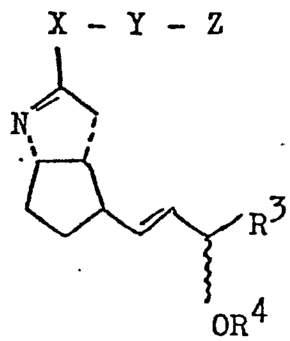

(I)

in welcher bedeuten

X ein Sauerstoff- oder Schwefelatom oder eine NH-Gruppe;

Y einen geradkettigen oder verzweigten Alkylenrest mit bis zu 8 Kohlenstoffatomen oder einen geradkettigen oder verzweigten ungesättigten aliphatischen Rest mit 3 bis 8 Kohlenstoffatomen oder einen cycloaliphatischen Rest mit 3 bis 6 Kohlenstoffatomen oder einen Phenylenrest;

Z einen Rest der Formel $-CO_2R^1$, $-CH_2OH$ oder $CH_2-N(R^2)_2$ wobei bedeuten

$R^1$ Wasserstoff oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 6 Kohlenstoffatomen oder einen cycloaliphatischen Kohlenwasserstoffrest mit 3 bis 7 Kohlenstoffatomen oder einen araliphatischen Kohlenwasserstoffrest mit 7 bis 9 Kohlenstoffatomen, oder ein physiologisch verträgliches Metall-, $NH_4$- oder ein Ammoniumion, das sich von einem primären, sekundären oder tertiären Amin ableitet, oder ein Tetraalkylammonium-Ion,

$R^2$ Wasserstoff oder einen geradkettigen oder verzweigten aliphatischen Kohlenwasserstoffrest mit bis zu 5 C-Atomen oder $R^2$-$R^2$ zusammen auch eine $-(CH_2)_n$-Gruppe mit n = 3 bis 6

$R^3$ einen Arylrest, der im Kern 1- bis 3-fach substituiert sein kann mit Halogen, Trifluormethyl und/oder Alkyl oder Alkoxy mit je 1 bis 6 C-Atomen, oder einen cycloaliphatischen Rest mit 3 bis 8 Kohlenstoffatomen oder einen geradkettigen oder verzweigten Alkylrest mit bis zu 8 Kohlenstoffatomen oder einen geradkettigen oder verzweigten ungesättigten aliphatischen Kohlenwasserstoffrest mit 3 bis 8 Kohlenstoffatomen, wobei Alkyl- und aliphatischer Kohlenwasserstoffrest ihrerseits substituiert sein können mit

a) einem geradkettigen oder verzweigten Alkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Phenyl oder einem α- oder ß-Thienyl- oder α- oder ß-Furylrest, die ihrerseits im Kern 1- bis 3-fach substituiert sein können mit Halogen, Trifluormethyl und/oder Alkyl oder Alkoxy mit je 1 bis 6 C-Atomen,

c) einem Phenoxy-, einem α- oder ß-Thienyloxyrest oder einem Cycloalkoxyrest mit 3 bis 7 Kohlenstoffatomen, wobei die genannten Reste ihrerseits im Kern 1- bis 3-fach substituiert sein können mit Halogen, Trifluormethyl und/oder Alkyl oder Alkyloxy mit je bis 6 C-Atomen,

$R^4$ Wasserstoff oder eine unter neutralen oder basischen Bedingungen leicht abspaltbare Schutzgruppe, dadurch gekennzeichnet, daß man

a) eine Verbindung der Formel XIV oder XV

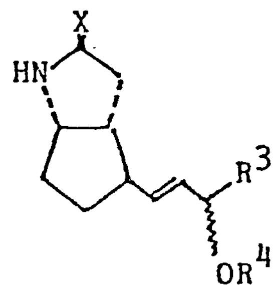

(XIV)
(XV)

worin $R^3$ die oben genannte Bedeutung hat, $R^4$ eine unter neutralen oder basischen Bedingungen leicht abspaltbare Schutzgruppe darstellt und X ein Sauerstoff- oder Schwefelatom bedeutet, mit einem Alkylhalogenid der Formel XVI

$$Z - Y - Hal \qquad (XVI)$$

worin Hal Jod, Chlor oder Brom bedeutet und Y und Z die oben genannten Bedeutungen haben, alkyliert zu einer Verbindung der Formel I, worin X ein Sauerstoff- oder Schwefelatom darstellt, oder

b) eine Verbindung der Formel XVII

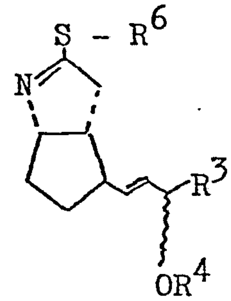

(XVII)

worin $R^3$ und $R^4$ die oben genannten Bedeutungen haben und $R^6$ einen $C_{1-4}$-Alkylrest bedeutet, mit einem Amin der Formel XVIII

$$Z - Y - NH_2 \qquad (XVIII)$$

worin Z und Y die oben genannten Bedeutungen haben, umsetzt zu einer Verbindung der Formel I, worin X den NH-Rest bedeutet,

c) gegebenenfalls in einer Verbindung der Formel I die Schutzgruppe $R^4$ durch basisch katalysierte Hydrolyse abspaltet,

d) gegebenenfalls eine Verbindung der Formel I, in welcher Z den Rest $CO_2R^1$ bedeutet, wobei $R^1$ den oben genannten Rest jedoch nicht Wasserstoff oder ein Kation darstellt und $R^4$ Wasserstoff bedeutet, zu einer Verbindung der Formel I verseift, in welcher Z den Rest $CO_2R^1$ darstellt, wobei $R^1$ Wasserstoff oder ein physiologisch verträgliches Kation bedeutet,

e) gegebenenfalls eine Verbindung der Formel I, in welcher Z den Rest $CO_2R^1$ darstellt, wobei $R^1$ Wasserstoff oder ein Kation bedeutet, zu einer Verbindung der Formel I verestert, worin Z den Rest $CO_2R^1$ darstellt, wobei $R^1$ die zur Formel I genannten Bedeutungen hat, jedoch nicht Wasserstoff oder ein Kation bedeutet, und

f) gegebenenfalls in einer Verbindung der Formel I, in welcher Z den Rest $CO_2R^1$ darstellt, wobei $R^1$ ein physiologisch verträglicher Kation darstellt, dieses Kation gegen ein anderes austauscht.

2. Verfahren zur Herstellung von Arzneimitteln, dadurch gekennzeichnet, daß man eine Verbindung der Formel I, gegebenenfalls mit üblichen pharmazeutischen Trägern und/oder Stabilisatoren, in eine therapeutisch geeignete Dareichungsform bringt.

**0055851**

Nummer der Anmeldung

Europäisches Patentamt

# EUROPÄISCHER RECHERCHENBERICHT

EP 81 11 0772.1

| Kategorie | EINSCHLÄGIGE DOKUMENTE<br>Kennzeichnung des Dokuments mit Angabe, soweit erforderlich, der maßgeblichen Teile | betrifft Anspruch | KLASSIFIKATION DER ANMELDUNG (Int Cl.³) |
|---|---|---|---|
| P,A | EP - A1 - 0 034 778 (HOECHST AG)<br>* Anspruch 2 *<br>-- | 2 | C 07 D 209/52<br>A 61 K 31/40<br>//C 07 D 409/06<br>C 07 D 409/12 |
| A | ANGEWANDTE CHEMIE, INT. ED.<br>Band 19, Nr. 10,<br>1980, VERLAG CHEMIE, Weinheim<br>W. BARTMANN et al. "New Prostacyclin Analogues"<br>Seiten 819 bis 820<br>---- | | |
| | | | RECHERCHIERTE SACHGEBIETE (Int. Cl.³)<br>A 61 K 31/40<br>C 07 D 209/52 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 30-03-1982 | FROELICH |